# EUROPEAN PATENT SPECIFICATION

(11) **EP 2 588 043 B1**
(45) Date of publication and mention of the grant of the patent: **25.02.2015**
(21) Application number: 11732530.8
(22) Date of filing: 01.07.2011
(51) Int. Cl.: A61F 5/01, A61F 5/042, A61F 5/048

(54) **ACHILLES TENDON STRETCHING DEVICE**
VORRICHTUNG ZUR STRECKUNG DER ACHILLESSEHNE
DISPOSITIF D'ÉTIRAGE DU TENDON D'ACHILLE

(30) Priority: 01.07.2010 US 360561 P; 17.03.2011 US 201161453633 P
(43) Date of publication of application: 08.05.2013
(73) Proprietor: Össur HF, 110 Reykjavik (IS)
(72) Inventor: INGIMUNDARSON, Arni, Thor, Foothill Ranch, CA 92610 (US); KRISTJANSSON, Arnar, Foothill Ranch, CA 92610 (US); EINARSSON, Palmi, Foothill Ranch, CA 92610 (US); GUNNSTEINSSON, Larus, Foothill Ranch, CA 92610 (US); INGVARSSON, Thorvaldur, 600 Akureyri (IS)
(74) Representative: Klunker . Schmitt-Nilson . Hirsch
(86) International application number: PCT/US2011/001168
(87) International publication number: WO 2012/003000

(56) References cited:
- WO-A1-92/04880
- WO-A1-2004/009001
- DE-U1- 9 314 920
- US-A- 5 902 259
- US-A1- 2009 287 127

## Description

### FIELD OF THE INVENTION

The present invention relates generally to the field of orthopedic or prosthetic devices and more particularly to a device for use in the treatment, repair, and rehabilitation of the Achilles tendon following an injury and/or corrective surgery.

### BACKGROUND

Achilles tendon rupture is the most common injury involving a tear in a tendon. It commonly occurs as a sports injury during explosive acceleration, for example, while pushing off or jumping up.

Treatment of Achilles tendon rupture is typically divided between operative and non-operative management, each of which involve the gradual stretching of the tendon after the rupture has healed.

Operative management involves a surgical operation where the ruptured tendon is sutured back together at the point of rupture, and the leg is then placed into a cast. When the leg is placed in the cast, the foot is pointed downward (in an equinus position). As the healing progresses, the equinus position is then gradually decreased (requiring removal of the original cast, and recasting with the newly decreased equinus position).

Non-operative management typically involves wearing a cast or walking boot, which allows the ends of torn tendon to reattach themselves on their own. In the non-operative option, the foot is pointed downwards, with the help of heel wedges or insoles, which are placed in the walker boot. The height of the heel wedges or insoles is then incrementally decreased as the process of healing progresses. One disadvantage of using the heel wedges is that reducing the height of the wedges/insole can be too drastic for the injured tendon.

Both the operative and the non-operative methods involve a long rehabilitation process, lasting at least 6 months. Additionally, in each situation, removal of the cast or walking boot is necessary in order to adjust the equinus position of the foot, and/or to remove and replace heel wedges or insoles having a different height.

Non-operative treatment might seem like a more comfortable way to go about Achilles repair, but it does take longer and leaves the patient with a greater risk of re-injury. Immobilization using a plaster cast can take as long as 12 months for the tendon to return to full strength, whereas surgery may only require anywhere for 6 to 9 months. In the past, patients who underwent surgery would wear a cast for approximately 4 to 8 weeks after surgery and were only allowed to gently move the ankle once out of the cast. Recent studies have shown that patients have quicker and more successful recoveries when they are allowed to move and lightly stretch their ankle immediately after surgery. To keep their ankle safe these patients use a removable boot while walking and doing daily activities.

In either the operative or the non-operative situation, existing methods for stretching the Achilles tendon can be time consuming and inconvenient to implement. Additionally, existing methods for stretching the Achilles tendon can cause drastic of changes in stretching the length of the Achilles tendon, such that the risk of re-rupture of the tendon is increased.

Regarding the prior art, WO 92/04880 A1 discloses a mechanism for putting the ankle at an angle relative to the longitudinal axis of the footwear, whereas DE 93 14 920 U1 describes a split shell walker boot including a foot bed for a foot.

### SUMMARY

In view of the above discussion, exemplary embodiments of an Achilles tendon stretching device are disclosed that provide improved mechanisms to allow more even stretching of the Achilles tendon, to reduce problems associated with edema, and to promote faster healing of the injured tendon in order to allow for faster recovery and shorter rehabilitation times.

Exemplary embodiments of an Achilles tendon stretching device can include manual or automatic mechanisms to allow incremental height changes to an insole placed within a walking boot, orthopedic shoe, or post surgical shoe, in order to allow more even and gradual stretching of the Achilles tendon.

Exemplary mechanisms can include manual or automatic screw mechanisms, as well as manual or automatic pneumatic systems. Manual or automatic hydraulic systems are also contemplated.

Other exemplary mechanisms can include the use of a heel wedge having layers which can be torn or cut off to adjust the height of the heel wedge.

By utilizing the disclosed exemplary embodiments of an Achilles tendon stretching device, the degree of stretching of the tendon can be more easily, and more evenly controlled, in order to avoid drastic changes in the stretched length of the tendon, which may lead to re-rupture of the tendon.

### BRIEF DESCRIPTION OF THE DRAWINGS

These and other features, aspects, and advantages of the present invention will become better understood with regard to the following description, appended claims, and accompanying drawings where:
Fig. 1 is a perspective view of a circumferential type walking boot (walker) in which the exemplary embodiments of an Achilles tendon stretching device may be implemented;
Fig. 2 is a side view of another type of walker in which the exemplary embodiments of an Achilles tendon stretching device may be implemented;
Fig. 3 is a rear perspective view of a first exemplary embodiment of an Achilles tendon stretching device implemented in a circumferential type walker;
Fig. 4 represents a partial cut-away side view of the exemplary embodiment of an Achilles tendon stretching device shown in Fig. 3;
Fig. 5 represents a partial cut-away side view of another exemplary embodiment of an Achilles tendon stretching device;
Fig. 6 represents a partial cut-away side view of another exemplary embodiment of an Achilles tendon stretching device;
Fig. 7 represents a partial cut-away side view of another exemplary embodiment of an Achilles tendon stretching device;
Fig. 8 represents a partial cut-away side view of another exemplary embodiment of an Achilles tendon stretching device;
Fig. 9 represents a perspective view of another exemplary embodiment of an Achilles tendon stretching device in the form of a heel wedge having layers which can be torn or cut off to adjust the height of the heel wedge; and
Fig. 10 is a partial exploded side view of the Achilles tendon stretching device shown in Fig. 9.

### DETAILED DESCRIPTION

### A. Environment and Context

Exemplary embodiments of an Achilles tendon stretching device are provided for use in the treatment, repair, and rehabilitation of the Achilles tendon following an injury and/or corrective surgery. Features that are provided on one side of the device can easily be provided on the other side of the device. In this manner, it is intended that the exemplary embodiments of the Achilles tendon stretching device described herein may be used on either right or left lower legs, with any appropriate reconfiguration of components that is deemed necessary for the proper fit and function of the device for the purpose of treatment, repair, and rehabilitation of the Achilles tendon of either the left or right lower leg.

In the exemplary embodiments of the Achilles tendon stretching device described herein, quick release strap mechanisms may be used to provide ease of securing and tightening the device to the lower leg. Exemplary quick release strap mechanisms are described in U.S. patent no. 7,198,610, granted April 2007, commonly owned.

The exemplary embodiments of the disclosure are adapted for treatment, repair, and rehabilitation of the Achilles tendon of human beings, and may be dimensioned to accommodate different types, shapes and sizes of human joints and appendages.

The exemplary embodiments of an Achilles tendon stretching device can be implemented in various configurations of walking boots, orthopedic shoes, or post surgical shoes.

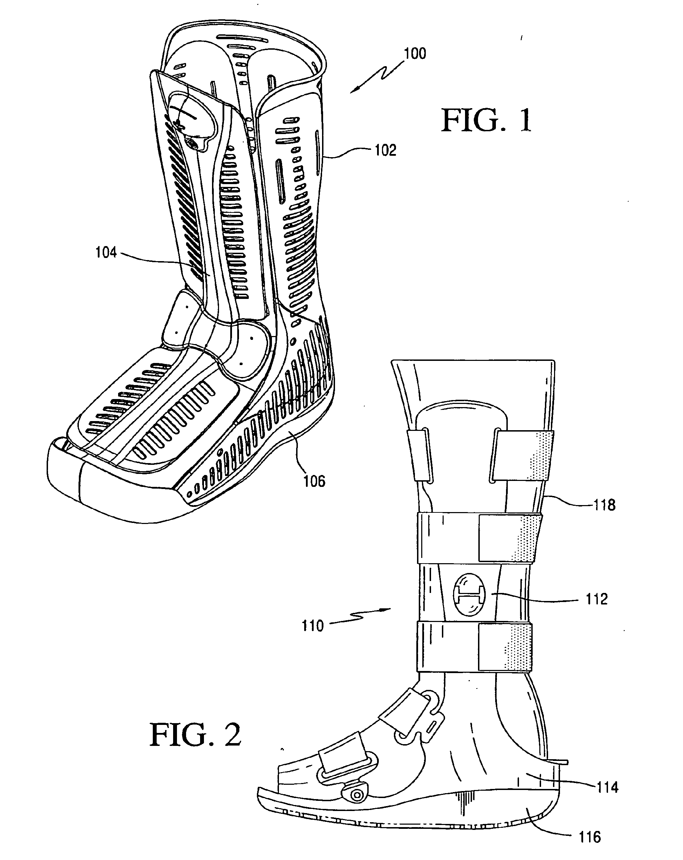

For example, exemplary embodiments of an Achilles tendon stretching device can be implemented within a circumferential type walker 100, as shown in Fig. 1. An exemplary circumferential type walker 100 includes a posterior shell 102 and an anterior, dorsal shell or plate 104, such that the lower leg is generally fully enclosed and supported by the walker 100. An outsole 106 is provided along the distal plantar surface of the walker 100.

Further, exemplary embodiments of an Achilles tendon stretching device can be implemented within a walker 110, as shown in Fig. 2. The walker 110 includes a sole portion 114 having supporting struts 112 extending therefrom, and an outsole 116. A liner 118 is provided enclosing the lower leg and positioned between and supported by the supporting struts 112.

Exemplary materials and configurations for components of the Achilles tendon stretching device, such as sole portions and shell portions, are described in detail in U.S. patent nos. 5,078,128, granted January 1992, 5,329,705, granted July 1994, 5,464,385, granted November 1995, and 7,303,538, granted December 2007, all commonly owned. Additional exemplary materials and configurations for components of the Achilles tendon stretching device can be found in U.S. publication no. 2009/0287127, published November 19, 2009, commonly owned.

For further ease of understanding the exemplary embodiments of an orthopedic device as disclosed herein, a description of a few terms is necessary. As used herein, the term "dorsal" has its ordinary meaning and refers to the top surfaces of the foot, ankle and foreleg or shin. As used herein, the term "plantar" has its ordinary meaning and refers to a bottom surface, such as the bottom of a foot. As used herein, the term "proximal" has its ordinary meaning and refers to a location that is closer to the heart than another location. Likewise, the term "distal" has its ordinary meaning and refers to a location that is further from the heart than another location. The term "posterior" also has its ordinary meaning and refers to a location that is behind or to the rear of another location. Lastly, the term "anterior" has its ordinary meaning and refers to a location that is ahead of or to the front of another location.

The terms "rigid," "flexible," and "resilient" may be used herein to distinguish characteristics of portions of certain features of the orthopedic device. The term "rigid" is intended to denote that an element of the device is generally devoid of flexibility. Within the context of support members or shells that are "rigid," it is intended to indicate that they do not lose their overall shape when force is applied, and in fact they may break if bent with sufficient force. On the other hand, the term "flexible" is intended to denote that features are capable of repeated bending such that the features may be bent into retained shapes or the features do not retain a general shape, but continuously deform when force is applied. The term "resilient" is used to qualify such flexible features as generally returning to an initial general shape without permanent deformation. As for the term "semi-rigid," this term is used to connote properties of support members or shells that provide support and are free-standing, however such support members or shells may have some degree of flexibility or resiliency.

### B. First Exemplary Embodiment

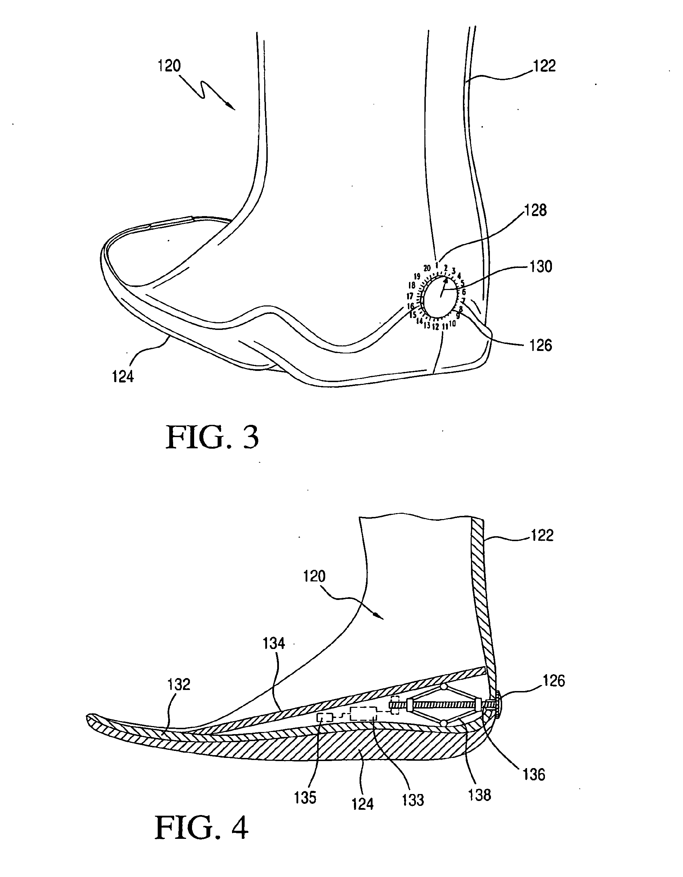

A first exemplary embodiment of an Achilles tendon stretching device is shown in Figs. 3 and 4 as implemented in a generally circumferential walker 120 of the type shown in Fig. 1.

As shown, the walker 120 includes a posterior shell 122 and an outsole 124. An adjustment dial 126 is provided to allow adjustment of the height of an insole 134 in order to adjust the amount of stretching of the Achilles tendon.

In order to aid with determining the amount of stretching of the Achilles tendon, a marker 130 is provided on the adjustment dial 126 which points at distinct indicia 128, for example numerical indicators, provided on the posterior shell 122 and/or the outsole 124. It will be recognized that the indicia may be provided on the adjustment dial 126 itself, and the marker may be provided on the posterior shell 122 or the outsole 124.

In order to provide a more even and less drastic stretching, each numerical indication of indicia 128 can represent a height change (increase or decrease) of 1 mm of the insole 134, such that each incremental movement of the adjustment dial 126 from one indicia to the next represents a change in height of the insole 134 of 1 mm. Of course, the height change represented by the incremental rotation of the adjustment dial 126 may be more or less than 1 mm.

As shown in Fig. 4, the adjustment dial 126 includes an adjustment screw 136 that can be connected to a scissor jack mechanism 138 positioned between the insole 134 and a plantar sole portion 132.

The adjustment dial 126 can be manually rotated, and may include appropriate textures or shapes to aid with manual manipulation such as gripping thereof by the person adjusting the mechanism.

In the exemplary configuration, with manual adjustment of the adjustment dial 126, a practitioner or other authorized person can rotate the adjustment dial 126 one increment, once a week, in order to decrease the height of the insole 134 by 1 mm once every week in order to increase the amount of stretch of the Achilles tendon by 1 mm each week during use of the device. In this manner, a more even stretch of the Achilles tendon can be achieved, in order to avoid drastic changes in the stretched length of the tendon, which may lead to re-rupture of the tendon. With the exemplary configuration of the Achilles tendon stretching device, the walker 120 does not need to be removed from the patient in order to achieve adjustment thereof.

In a variation, an electric motor 133 (shown in outline in Figure 4) may be provided, along with an appropriate control mechanism, in order allow for automatic adjustment of the adjustment dial 126. Such an adjustment may be made once a week, as discussed above, or may be accomplished in even smaller increments throughout a week in order to provide an even more gradual change in the amount of stretching of the Achilles tendon.

Appropriate onboard electronics (including, for example, sensors such as accelerometers, strain gauges, angle sensors, gyroscopes, etc.) and communication mechanisms (such as wireless Internet, Wi-Fi, Bluetooth, infrared, etc.) can be provided 135 (exemplarily shown in outline in Figure 4) in order to sense the amount of activity of the user (and healing of the Achilles tendon), to automatically adjust the height of the insole 134 appropriately, and to provide communicative messages to a practitioner who may then remotely monitor the progress of the healing of the Achilles tendon.

In another variation of the Achilles tendon stretching device, instead of the adjustment dial 126, a socket head cap screw (or other suitable machine screw) can be provided as the adjustment screw 136.

### C. Second Exemplary Embodiment

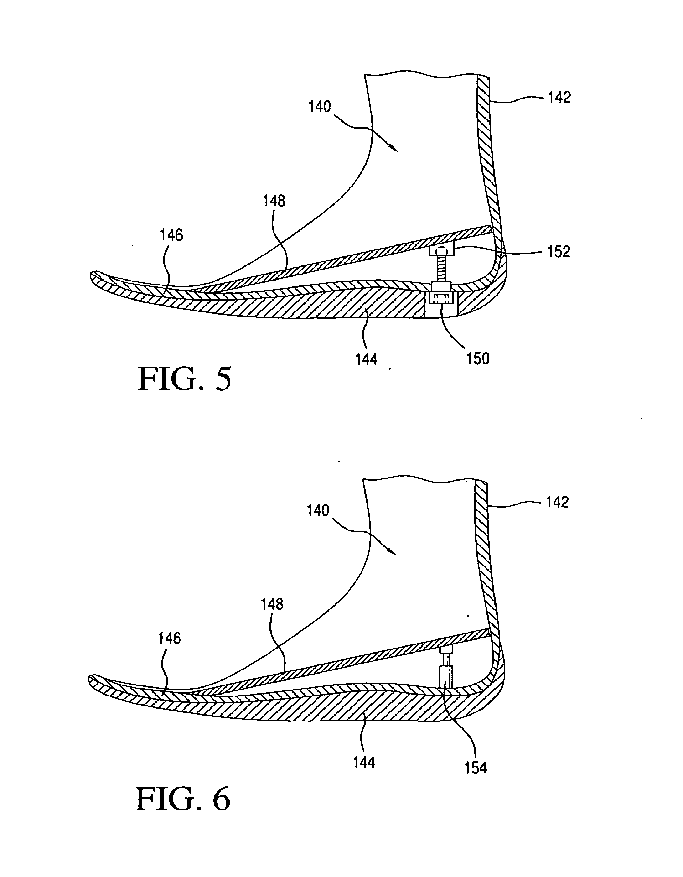

In a second exemplary embodiment of an Achilles tendon stretching device shown in Fig. 5, a generally circumferential walker 140 has a posterior shell 142, a planter sole 146, and an outsole 144.

In contrast to the embodiment shown in Figs. 3 and 4, the adjustment mechanism is provided through the plantar sole 146 and the distal surface of the outsole 144. Additionally, instead of an adjustment dial, a socket head cap screw (or other suitable machine screw) is provided as the adjustment screw 150. The practitioner can utilize an Allen wrench in order to adjust the height of the insole 148 in order to adjust the amount of stretching of the Achilles tendon.

The far end of the adjustment screw 150 can be connected to the insole 148 by way of a universal joint 152, which allows rotation of the end of the adjustment screw 150, while providing a vertical translation of the insole 148.

Instead of the socket head cap screw, a dial with indicia, similarly to adjustment dial 126, may be used.

Rotation of the adjustment screw 150 to decrease the height of the insole 148 will cause the socket head portion of the adjustment screw 150 to become spaced from the plantar sole 146. The depth of the opening in the outsole 144 should be sufficient to accommodate the movement of the socket head portion of the adjustment screw 150.

Alternatively, an appropriate mechanical mechanism can be provided to cause the rotation of the adjustment screw 150 to be translated into the vertical height adjustment of the insole 148, without any corresponding vertical movement of the adjustment screw 150, such as for example, a scissor jack mechanism.

Similarly as discussed above, an electric motor and associated control mechanisms can be provided in order to allow automatic (gradual and/or incremental) adjustment of the adjustment screw 150.

In a variation shown in Figure 6, the adjustment screw 150 may be replaced by a pneumatic or hydraulic piston and cylinder 154, which may be controlled in a known manner to achieve vertical height adjustment of the insole 148 with respect to the plantar sole 146.

Like the previously discussed embodiment, a practitioner can adjust this Achilles tendon stretching device once a week, in order to decrease the height of the insole 148 by 1 mm once every week in order to increase the amount of stretch of the Achilles tendon by 1 mm each week during use of the device. In this manner, a more even stretch of the Achilles tendon can be achieved, in order to avoid drastic changes in the stretched length of the tendon, which may lead to re-rupture of the tendon. Similarly, with this exemplary configuration of the Achilles tendon stretching device, the walker 140 does not need to be removed from the patient in order to achieve adjustment thereof.

### D. Third Exemplary Embodiment

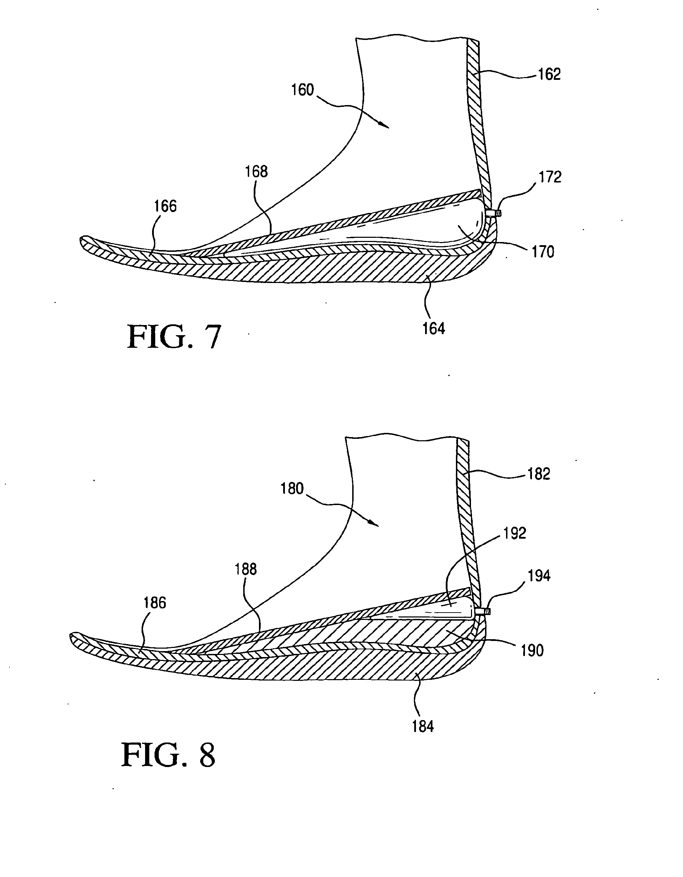

A third exemplary embodiment of an Achilles tendon stretching device is shown in Fig. 7. In this embodiment, a walker 160 includes a posterior shell 162, a plantar sole 166, an outsole 164, and an adjustable height insole 168.

A pneumatic bladder 170 constructed of suitable materials capable of retaining a gas such as air therein, is provided between the plantar sole 166 and the insole 168. A valve mechanism 172 is provided at the posterior of the bladder 170 and protruding through an opening in the posterior shell 162 for access thereto by a practitioner. Any suitable valve mechanism, such as, for example a Schrader valve or a Presta valve, can be provided. It will be recognized that the valve mechanism 172 can alternatively be positioned to protrude through the plantar sole 166 and outsole 164.

Once a week, the patient can visit the practitioner, and the practitioner can release an appropriate amount of gas from the bladder 170, using the valve 172, in order to decrease the height of the insole 168 by an appropriate amount, for example, 1 mm.

Like the previously discussed embodiments, the walker 160 need not be removed from the patient in order to effectuate adjustment of the height of the insole 168. Similarly, a more even stretch of the Achilles tendon can be achieved, in order to avoid drastic changes in the stretched length of the tendon, which may lead to re-rupture of the tendon.

As a variation, a slow leak valve can be provided to automatically and continuously release a predefined amount of gas from the bladder 170 in order to automatically and gradually reduce the height of the insole 168, for example, an amount of 1 mm per week. Such a slow leak valve can be, for example, a slit valve designed to allow the slit to partially open in response to a continuous predefined pressure applied by the weight of the wearer of the walker 160. Alternatively, the slow leak valve can be any mechanical valve that can be set to an open or closed configuration, and which can be set in a slightly opened position to allow the slow leak. For example, any suitable ball valve, disc valve, butterfly valve, etc., may be utilized as a slow leak valve.

As a further variation, appropriate processor and automatic valve (for example an electrically operated solenoid valve) mechanisms can be provided to effectuate gradual and/or incremental release of gas from the bladder 170.

In yet another variation, as shown in Fig. 8, a walker 180 includes a posterior shell 182, a plantar sole 186, an outsole 184, an adjustable height insole 188, and a bladder 192 having a valve 194. This variation of the Achilles tendon stretching device functions in generally the same manner as the device shown in Fig. 6, with the exception that in addition to the bladder 192 positioned between the plantar sole 186 and the insole 188, removable and interchangeable heel wedges 190 having different heights are provided (one at a time) between the bladder 192 and the insole 188 and the plantar sole 186. Alternatively, multiple heel wedges of consistent heights can be provided in place of the interchangeable heel wedges 190.

With this configuration and an automatically controlled release of gas from the bladder 192, the height of the insole 188 would gradually decrease throughout a week, and when the patient visits the practitioner, the heel wedge 190 can be replaced with a heel wedge of lower height (or one of the multiple heel wedges can be removed) and the bladder 192 can be reinflated.

While pneumatic bladders and valves are disclosed, it is contemplated that hydraulic bladders and valves may also be used.

Again, these configurations provide for a more even stretch of the Achilles tendon can be achieved, in order to avoid drastic changes in the stretched length of the tendon, which may lead to re-rupture of the tendon.

### E. Fourth Exemplary Embodiment

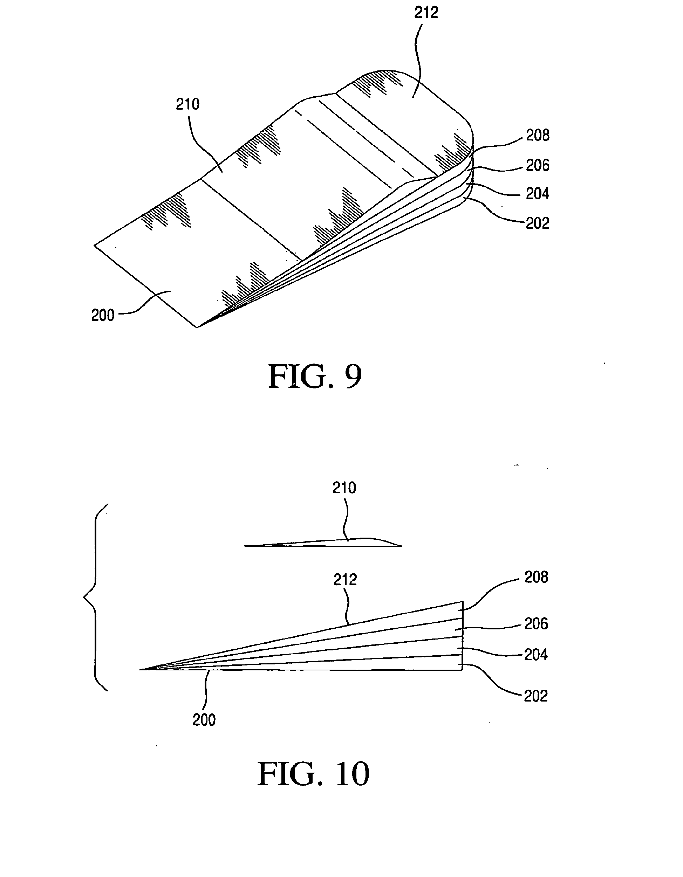

A fourth exemplary embodiment of an Achilles tendon stretching device in the form of a heel wedge 200 having layers 202, 204, 20, 208 which can be torn or cut off to adjust the height of the heel wedge is shown in Figs. 9 and 10.

The heel wedge 200 can be used in any orthopedic device for use with the foot, for example, walkers, diabetic walkers, post-op shoes, ankle braces, or any type of footwear, such as shoes or boots.

The heel wedge 200 can be made from any suitable material, for example ethylene-vinyl acetate (EVA) foam. Further, the heel wedge 200 can be made from compression molded EVA foam. Exemplary EVA foam can have a density/hardness in the range of 35-60 shore. Another suitable material may be an artificial cork, such as an EVA cork mixture that is thermo moldable at approximately 120 to 140 degrees and results in a density/hardness of 50 shore. Another exemplary material may be polyurethane.

As shown in Figs. 9 and 10, the heel wedge 200 has a number of layers, all of which are integrally formed and connected together at an anterior portion of the heel wedge 200. As shown the heel wedge 200 includes first layer 202, second layer 204, third layer 206, and fourth layer 208. However, the number of layers shown is merely exemplary, and any suitable greater or lesser number of layers, for example ten to fifteen layers, may be used in order to achieve the desired amount and increments of height adjustment.

In use, the heel wedge 200 can be positioned within the heel portion of orthopedic device with all of the integrally formed layers thereof retained. Thus, the Achilles tendon of a user of the heel wedge 200 will be shortened to a first length.

In order to incrementally stretch the Achilles tendon of the user of the heel wedge 200, the layers 202, 204, 206, 208 thereof can be incrementally cut or torn from the heel wedge 200 in order to reduce the height of the heel wedge 200, thus stretching the Achilles tendon of the user to incrementally greater lengths.

The thickness or height of the layers 202, 204, 206, 208 corresponds to the desired incremental stretch length of the Achilles tendon, and may be any desired thickness or height.

In use, to adjust the stretch length of the Achilles tendon at a first time, the heel wedge 200 can be removed from the heel portion of orthopedic device. Then, the first layer 202 can be torn or cut away from the heel wedge 200, which can then be replaced in the heel portion of orthopedic device.

Treatment can then occur for the desired length of time to stretch the Achilles tendon at the length that is provided by removing the first layer 202 of the heel wedge 200. This process can be repeated as necessary by removing subsequent layers 204, 206, 208 in succession to treat Achilles tendon injuries and surgical recovery with incremental stretching of the Achilles tendon.

It is noted that if greater height adjustment is needed during a specified treatment period, more than one of the adjacent layers 202, 204, 206, 208 can be removed simultaneously. For example, after an initial treatment period using the heel wedge 200 having layers 202, 204, 206, 208, the adjacent first and second layers 202, 204 can be torn off of cut away in order to provide a greater height adjustment to increase the amount that the Achilles tendon is stretched.

This process can allow the use of a heel wedge 200 having numerous layers of minimal thickness, for example 1 mm, for numerous treatment therapies for Achilles tendon injuries. Such a heel wedge and process can then be modified for each particular treatment protocol, where, for example one protocol requires an incremental height adjustment of 1 mm per week, and another treatment protocol requires an incremental height adjustment of 5 mm per week.

For the treatment protocol requiring an incremental height adjustment of 1 mm per week, only a single layer of the heel wedge 200 would be removed each week. Similarly, for the treatment protocol requiring an incremental height adjustment of 5 mm per week, five of the layers of the heel wedge 200 would be removed each week.

Thus, in this manner, only one type of heel wedge 200 need be manufactured in order to satisfy numerous treatment protocols.

In order to provide additional stability to the Achilles tendon, and to prevent slipping of the user's heel within the orthopedic device and, therefore inadvertent stretching of the Achilles tendon, a heel stop 210 can be provided on the proximal surface 212 of the heel wedge 200.

The heel stop 210 can be formed, for example, from a compression molded EVA foam, or can be an injected molded thermoplastic elastomer (TPE), or any other suitable material.

As seen in Figs. 9 and 10, the heel stop 210 can have a generally triangular shape, with a raised ridge provided on a proximal surface thereof, such that the raised ridge provides a stop against forward migration of the user's heel during use. In an alternative configuration, the heel stop 210 can have a generally trapezium or trapezoidal shape, such that a planar surface is provided at the raised ridge. The planar surface can thus provide an additional frictional surface to engage at least a portion of the arch of the user's foot in order to prevent slippage of the user's heel.

The heel stop 210 can be glued or secured with adhesive directly to the proximal surface 212 of the heel wedge 200. Alternatively, the heel stop 210 may be held in place on the proximal surface 212 of the heel wedge 200 by the weight of the user and frictional forces.

Thus, it can be seen that the heel wedge 200 having a number of layers, all of which are integrally formed and connected together at an anterior portion of the heel wedge 200, and which can be selectively removed as desired to achieve incremental height adjustment and stretching of the Achilles tendon provides a convenient and simple way to effect treatment and recovery for Achilles tendon injuries and surgeries.

### F. Conclusion

It will be recognized that the exemplary embodiments of an Achilles tendon stretching device and components thereof can be made from any suitable materials.

While one week has been described above as a suitable time period for measuring when adjustment to the Achilles tendon stretching device should be made, it will be recognized that any suitable or desired time period may be utilized.

It will also be recognized that the various locations of each of the adjustment mechanisms of the exemplary Achilles tendon stretching devices described herein can be variously located in any convenient location, for example, at the posterior of the walker or in the plantar sole region of the walker.

While specific mechanical mechanisms (for example, a scissor jack or a socket head cap screw and universal joint) are described herein for adjusting the height of an insole with respect to the planter sole of a walker, any suitable mechanical, pneumatic, and/or hydraulic mechanism can be used to adjust the height of the insole. For example, pneumatic or hydraulic cylinders and pistons may be positioned between the insoles and plantar soles and used to raise and lower the adjustable height insoles. For example, a pneumatic or hydraulic cylinder and piston can be oriented to provide vertical translation to raise or lower the insole with respect to the plantar sole. Such pneumatic or hydraulic cylinders and pistons can be configured to automatically adjust height, either incrementally or continuously, as discussed in detail above with respect to alternatively recited configurations. The specific design and implementation of such pneumatic or hydraulic cylinders and pistons will be recognized by a person having skill the art.

## Claims

1. An orthopedic device for use as an Achilles tendon stretching device in a footwear, the device comprises said footwear (100, 110, 120, 140, 160, 180), **characterized in that**:
the footwear (100, 110, 120, 140, 160, 180) includes a height adjustable insole (134, 148, 168, 188, 212) and a plantar sole (132, 146, 166, 186), wherein a manual or automatic height adjustment mechanism (126, 136, 150, 154, 170, 192, 200) is provided to gradually and/or incrementally adjust the height of the insole (134, 148, 168, 188, 212) with respect to the plantar sole (126, 150, 154, 170, 192, 200).

2. The orthopedic device according to claim 1, wherein the height adjustment mechanism comprises:
an adjustment screw (136); and
an adjustment dial (126).

3. The orthopedic device according to claim 2, wherein the height adjustment mechanism further comprises:
a jack mechanism (138) positioned between the insole (134) and the plantar sole (132), wherein rotation of the adjustment dial (126) causes rotation of the adjustment screw (136) to raise or lower the jack mechanism (138) in order to adjust the height of the insole (134) with respect to the plantar sole (132).

4. The orthopedic device according to claim 2, wherein the adjustment dial (126) or the footwear (120) includes a marker (130), which points at distinct indicia (128) provided on the footwear (120) or the adjustment dial (126) in order to represent an incremental change in height of the insole (134) with respect to the plantar sole (132).

5. The orthopedic device according to claim 2, wherein the adjustment dial (126) is configured for manual rotation including textures or shapes to aid with manual manipulation thereof, or includes an electric motor (133) and a control mechanism to allow for automatic adjustment of the adjustment dial (126).

6. The orthopedic device according to claim 1, wherein the footwear (120) includes onboard electronics mechanisms (135) to sense a level of activity of a user and to automatically adjust the height of the insole (134) relative to the plantar sole (132) in response to the sensed activity level.

7. The orthopedic device according to claim 1, wherein the footwear (120) includes onboard communication mechanisms (135) configured to provide messages to a practitioner for monitoring progress a wearer of the orthopedic device.

8. The orthopedic device according to claim 1, the height adjustment mechanism comprises:
an adjustment screw (150) passing through the plantar sole (146) and connected to the insole (148) by a universal joint (152) to translate rotation of the adjustment screw (150) into vertical adjustment of the insole (148) with respect to the plantar sole (146).

9. The orthopedic device according to claim 1, wherein the height adjustment mechanism comprises:
a bladder (170) positioned between the insole (168) and the plantar sole (166), the bladder (170) having a valve (172) connected thereto for automatically and/or selectively releasing gas or fluid therefrom.

10. The orthopedic device according to claim 9, wherein the valve (172) is a slow leak valve configured to automatically and continuously release a predefined amount of gas or fluid from the bladder (170).

11. The orthopedic device according to claim 9, further comprising:
a processor, and
wherein the valve (172) is a solenoid valve controlled by the processor to provide gradual and/or incremental release of gas or fluid from the bladder (170).

12. The orthopedic device according to claim 9, further comprising:
removable and interchangeable heel wedges (190) having different heights provided in combination with the bladder (192) to adjust the height of the insole (188) with respect to the plantar sole (186).

13. The orthopedic device according to claim 9, further comprising:
removable heel wedges (190) having consistent heights provided in combination with the bladder (192) to adjust the height of the insole (188) with respect to the plantar sole (186).

14. The orthopedic device according to claim 1, wherein the height adjustment mechanism comprises:
a heel wedge (200) having a plurality of integrally formed layers (202-208) connected at an anterior portion thereof;
wherein a proximal surface (212) of the heel wedge (200) forms at least a portion of the insole, and the layers (202-208) of the heel wedge (200) can be selectively removed from the heel wedge (200) in order to adjust the height of the proximal surface (212) of the heel wedge (200) with respect to the plantar sole.

15. The orthopedic device according to claim 14, wherein the heel wedge (200) includes a heel stop (210) provided on the proximal surface (212) of the heel wedge (200).

## Patentansprüche

1. Orthopädische Vorrichtung zur Verwendung als eine Achillessehnen-Dehnungsvorrichtung in einer Fußbekleidung, wobei die Vorrichtung die Fußbekleidung (100, 110, 120, 140, 160, 180) umfasst, **dadurch gekennzeichnet, dass**:
die Fußbekleidung (100, 110, 120, 140, 160, 180) eine höheneinstellbare Einlegesohle (134, 148, 168, 188, 212) und eine Innensohle (132, 146, 166, 186) enthält, wobei ein manueller oder automatischer Höheneinstellmechanismus (126, 136, 150, 154, 170, 192, 200) vorgesehen ist, um graduell und/oder inkrementell die Höhe der Einlegesohle (134, 148, 168, 188, 212) bezüglich der Innensohle (126, 150, 154, 170, 192, 200) einzustellen.

2. Orthopädische Vorrichtung nach Anspruch 1, wobei der Höheneinstellmechanismus umfasst:
eine Einstellschraube (136) und
einen Einstellwähler (126).

3. Orthopädische Vorrichtung nach Anspruch 2, wobei der Höheneinstellmechanismus des Weiteren umfasst:
ein Hebemechanismus (138), der zwischen der Einlegesohle (134) und der Innensohle (132) positioniert ist, wobei eine Drehung des Einstellwählers (126) eine Drehung der Einstellschraube (136) bewirkt, um den Hebemechanismus (138) anzuheben oder abzusenken, um die Höhe der Einlegesohle (134) bezüglich der Innensohle (132) einzustellen.

4. Orthopädische Vorrichtung nach Anspruch 2, wobei der Einstellwähler (126) oder die Fußbekleidung (120) eine Markierung (130) enthält, die auf eine eindeutige Anzeige (128) zeigt, die auf der Fußbekleidung (120) oder dem Einstellwähler (126) bereitgestellt ist, um eine inkrementelle Veränderung der Höhe der Einlegesohle (134) bezüglich der Innensohle (132) zu repräsentieren.

5. Orthopädische Vorrichtung nach Anspruch 2, wobei der Einstellwähler (126) für eine manuelle Drehung eingerichtet ist, einschließlich Texturen oder Formen, um bei einer manuellen Manipulation desselben zu helfen, oder einen Elektromotor (133) und einen Steuermechanismus enthält, um eine automatische Einstellung des Einstellwählers (126) zu erlauben.

6. Orthopädische Vorrichtung nach Anspruch 1, wobei die Fußbekleidung (120) einen eingebauten Elektronikmechanismus (135) enthält, um ein Aktivitätslevel eines Benutzers zu ermitteln und die Höhe der Einlegesohle (134) relativ zu der Innensohle (132) als Reaktion auf das ermittelte Aktivitätslevel automatisch einzustellen.

7. Orthopädische Vorrichtung nach Anspruch 1, wobei die Fußbekleidung (120) einen eingebauten Kommunikationsmechanismus (135) enthält, der eingerichtet ist, zum Überwachen eines Fortschritts eines Trägers der orthopädischen Vorrichtung Nachrichten an einen Arzt bereitzustellen.

8. Orthopädische Vorrichtung nach Anspruch 1, wobei der Höheneinstellmechanismus umfasst:
eine Einstellschraube (150), welche durch die Innensohle (146) hindurch verläuft und mit der Einlegesohle (148) mittels eines Universalgelenks (152) verbunden ist, um eine Drehung der Einstellschraube (150) in eine vertikale Einstellung der Einlegesohle (148) bezüglich der Innensohle (146) zu übersetzen.

9. Orthopädische Vorrichtung nach Anspruch 1, wobei der Höheneinstellmechanismus umfasst:
eine zwischen der Einlegesohle (168) und der Innensohle (166) positionierte Blase (170), wobei die Blase (170) ein damit verbundenes Ventil (172) zum automatischen und/oder selektiven Ablassen von Gas oder Flüssigkeit daraus aufweist.

10. Orthopädische Vorrichtung nach Anspruch 9, wobei das Ventil (172) ein langsam entweichendes Ventil ist, das eingerichtet ist, automatisch und kontinuierlich eine vordefinierte Menge von Gas oder Flüssigkeit aus der Blase (170) abzulassen.

11. Orthopädische Vorrichtung nach Anspruch 9, des Weiteren umfassend:
einen Prozessor und
wobei das Ventil (172) ein von dem Prozessor gesteuertes Elektromagnetventil ist, um ein graduelles und/oder inkrementelles Ablassen von Gas oder Flüssigkeit aus der Blase (170) bereitzustellen.

12. Orthopädische Vorrichtung nach Anspruch 9, des Weiteren umfassend:
entfernbare und austauschbare Fersenkeile (190), die unterschiedliche Höhen aufweisen, bereitgestellt in Kombination mit der Blase (192), um die Höhe der Einlegesohle (188) bezüglich der Innensohle (186) einzustellen.

13. Orthopädische Vorrichtung nach Anspruch 9, des Weiteren umfassend:
entfernbare Fersenkeile (190), die einheitliche Höhen aufweisen, bereitgestellt in Kombination mit der Blase (192), um die Höhe der Einlegesohle (188) bezüglich der Innensohle (186) einzustellen.

14. Orthopädische Vorrichtung nach Anspruch 1, wobei der Höheneinstellmechanismus umfasst:
einen Fersenkeil (200), der eine Mehrzahl von integral gebildeten Schichten (202-208) aufweist, die an ihrem anterioren Teil verbunden sind,
wobei eine proximale Oberfläche (212) des Fersenkeils (200) zumindest einen Teil der Einlegesohle bildet und die Schichten (202-208) des Fersenkeils (200) selektiv von dem Fersenkeil (200) entfernt werden können, um die Höhe der proximalen Oberfläche (212) des Fersenkeils (200) bezüglich der Innensohle einzustellen.

15. Orthopädische Vorrichtung nach Anspruch 14, wobei der Fersenkeil (200) einen Fersenanschlag (210) enthält, der auf der proximalen Oberfläche (212) des Fersenkeils (200) bereitgestellt ist.

## Revendications

1. Dispositif orthopédique destiné à une utilisation sous la forme de dispositif pour étirer le talon d'Achille dans une chaussure, le dispositif comprenant ladite chaussure (100, 110, 120, 140, 160, 180), **caractérisé en ce que**
la chaussure (100, 110, 120, 140, 160, 180) inclut une semelle intérieure de hauteur réglable (134, 148, 168, 188, 212) et une semelle plantaire (132, 146, 166, 186), dans lequel un mécanisme de réglage de hauteur manuel ou automatique (126, 136, 150, 154, 170, 192, 200) est fourni pour régler de manière graduelle et incrémentale la hauteur de la semelle intérieure (134, 148, 168, 188, 212) par rapport à la semelle plantaire (126, 150, 154, 170, 192, 200).

2. Dispositif orthopédique selon la revendication 1, dans lequel le mécanisme de réglage de hauteur comprend :
une vis de réglage (136), et
un cadran de réglage (126).

3. Dispositif orthopédique selon la revendication 2, dans lequel le mécanisme de réglage de hauteur comprend en outre :
un mécanisme formant vérin (138) positionné entre la semelle intérieure (134) et la semelle plantaire (132), dans lequel la rotation du cadran de réglage (126) provoque la rotation de la vis de réglage (136) pour lever ou abaisser le mécanisme formant vérin (138), afin de régler la hauteur de la semelle intérieure (134) par rapport à la semelle plantaire (132).

4. Dispositif orthopédique selon la revendication 2, dans lequel le cadran de réglage (126) ou la chaussure (120) inclut un marqueur (130), qui pointe sur des repères distincts (128) prévus sur la chaussure (120) ou le cadran de réglage (126) afin de représenter une modification incrémentale de hauteur de la semelle intérieure (134) par rapport à la semelle plantaire (132).

5. Dispositif orthopédique selon la revendication 2, dans lequel le cadran de réglage (126) est configuré en vue d'une rotation manuelle incluant des textures ou formes afin d'aider sa manipulation manuelle, ou inclut un moteur électrique (133) et un mécanisme de commande afin de permettre un réglage automatique du cadran de réglage (126).

6. Dispositif orthopédique selon la revendication 1, dans lequel la chaussure (120) inclut des mécanismes électroniques embarqués (135) afin de détecter un niveau d'activité d'un utilisateur et de régler automatiquement la hauteur de la semelle intérieure (134) par rapport à la semelle plantaire (132) en réaction au niveau d'activité détecté.

7. Dispositif orthopédique selon la revendication 1, dans lequel la chaussure (120) inclut des mécanismes de communication embarqués (135) configurés pour fournir des messages à un praticien afin de surveiller les progrès d'un porteur du dispositif orthopédique.

8. Dispositif orthopédique selon la revendication 1, dans lequel le mécanisme de réglage de hauteur comprend :
une vis de réglage (150) traversant la semelle plantaire (146) et reliée à la semelle intérieure (148) par un joint universel (152) afin de translater la rotation de la vis de réglage (150) en un réglage vertical de la semelle intérieure (148) par rapport à la semelle plantaire (146).

9. Dispositif orthopédique selon la revendication 1, dans lequel le mécanisme de réglage de hauteur comprend :
une poche (170) positionnée entre la semelle intérieure (168) et la semelle plantaire (166), la poche (170) présentant une valve (172) reliée à celle-ci de manière à libérer de manière automatique et/ou sélective un gaz ou un fluide hors de celle-ci.

10. Dispositif orthopédique selon la revendication 9, dans lequel la valve (172) est une valve à fuite lente configurée pour libérer automatiquement et en continu une quantité prédéfinie de gaz ou fluide hors de la poche (170).

11. Dispositif orthopédique selon la revendication 9, comprenant en outre :
un processeur, et
dans lequel la valve (172) est une électrovalve commandée par le processeur afin de fournir une libération graduelle et/ou incrémentale de gaz ou de fluide hors de la poche (170).

12. Dispositif orthopédique selon la revendication 9, comprenant en outre :
des cales de talon amovibles et interchangeables (190) présentant des hauteurs différentes prévues en association avec la poche (192) afin de régler la hauteur de la semelle intérieure (188) par rapport à la semelle plantaire (186).

13. Dispositif orthopédique selon la revendication 9, comprenant en outre :
des cales de talon amovibles (190) présentant des hauteurs uniformes prévues en association avec la poche (192) afin de régler la hauteur de la semelle intérieure (188) par rapport à la semelle plantaire (186).

14. Dispositif orthopédique selon la revendication 1, dans lequel le mécanisme de réglage de hauteur comprend :
une cale de talon (200) présentant une pluralité de couches formées d'un seul tenant (202-208) reliées au niveau d'une partie antérieure de celles-ci ;
dans lequel une surface proximale (212) de la cale de talon (200) forme au moins une partie de la semelle intérieure, et les couches (202-208) de la cale de talon (200) peuvent être entièrement retirées de la cale de talon (200) afin de régler la hauteur de la surface proximale (212) de la cale de talon (200) par rapport à la semelle plantaire.

15. Dispositif orthopédique selon la revendication 14, dans lequel la cale de talon (200) inclut un arrêt de talon (210) fourni sur la surface proximale (212) de la cale de talon (200).
